# EUROPÄISCHE PATENTANMELDUNG

(11) **EP 1 559 379 A1**
(43) Veröffentlichungstag der Anmeldung: **03.08.2005**
(21) Anmeldenummer: 04018567.0
(22) Anmeldetag: 05.08.2004
(51) Int. Cl.: A61B 19/00

(54) **Vorrichtung zum Setzen von Markern in ein Gewebe**

(30) Priorität: 31.01.2004 DE 102004004966
(71) Anmelder: Labotect Verwaltungs GmbH, D-37079 Göttingen (DE)
(72) Erfinder: Emons, Günter, Prof. Dr., 37085 Göttingen (DE); Nia, Abdolhamid Huschmand, Dr., 37075 Göttingen (DE); Horvath, Tibor, 37085 Göttingen (DE)
(74) Vertreter: Rehberg Hüppe + Partner

(57) **Zusammenfassung**

Bei einer Vorrichtung zum *in vivo* Setzen von Markern in ein biologisches Gewebe mit einer ein offenes distales Ende (3) aufweisenden Applikatorkanüle (2), in der eine elastisch verformte Vorform (15) mindestens eines ringförmigen Markers aus Federdraht (22) angeordnet ist, und mit einer Betätigungseinrichtung, um die Vorform (15) aus dem distalen Ende (3) der Applikatorkanüle (2) auszustoßen, wobei sich die Vorform (15) beim Freiwerden aus der Applikatorkanüle (2) durch elastische Rückstellkräfte zu dem Marker rückformt, ist die Vorform (15) in der Applikatorkanüle (2) linear gestreckt, und die Spitze (14) der Vorform (15) beschreibt beim Freiwerden aus der Applikatorkanüle (2) einen tangential in das distale Ende (3) der Applikatorkanüle (2) einmündenden Kreisbogen (20).

## Beschreibung

Die Erfindung betrifft eine Vorrichtung zum *in vivo* Setzen von Markern in ein biologisches Gewebe mit den Merkmalen des Oberbegriffs des Patentanspruchs 1.

Der den Marker ausbildende Federdraht kann ein Federstahldraht aber auch ein federnder Draht aus einem anderen Material einschließlich anderen Metallen und auch Kunststoffen sein, der sich elastisch zu der Vorform verformen lässt und sich ohne äußere Kräfte aus der Vorform zu dem Marker zurückformt.

Das *in vivo* Setzen von Markern in ein biologisches Gewebe kann beispielsweise dazu dienen, den Bereich eines Tumors zu markieren, der anschließend einer Bestrahlung oder Chemotherapie unterworfen wird. Die Marker in dem Gewebe machen es nach der Bestrahlung bzw. Chemotherapie möglich, den Ort wiederzufinden, an dem sich der Tumor ursprünglich befunden hat, auch wenn er aufgrund der Behandlung in seiner Größe stark zurückgegangen oder gar soweit verschwunden ist, dass er mit den angewandten Diagnosemitteln ohne die Marker nicht wiedergefunden werden kann.

### STAND DER TECHNIK

Die WO 96/08208 offenbart eine Vielzahl von Vorrichtungen zum *in vivo* Setzen von Markern in ein biologisches Gewebe in unterschiedlichen Ausführungsformen. Eine Vorrichtung mit den Merkmalen des Oberbegriffs des Patentanspruchs 1 ist in der dortigen Figur 15 wiedergegeben. Ein ringförmiger Marker aus Federdraht, der einen Kreisbogen von etwas mehr als 360° überspannt, so dass seine Enden leicht überlappen, ist zu einer helikalen Vorform eng um seine Ringachse zusammengewickelt. Diese Vorform ist mit koaxialer Ausrichtung in einer Applikatorkanüle angeordnet, wobei die in der Vorform wirkenden radialen Rückstellkräfte von der die Vorform umschließenden Applikatorkanüle abgestützt werden. In der Kanüle ist ein mandrelartiger Kolben von dem proximalen Ende der Kanüle her vorschiebbar, um die Vorform des Markers aus dem distalen Ende der Applikatorkanüle auszustoßen. Beim Freiwerden der Vorform von der Applikatorkanüle weitet sich die helikale Vorform in radialer Richtung aus, bis sie die ringförmige Gestalt des Markers aufweist. Dabei wird das umgebende biologische Gewebe radial verdrängt. Eine Verankerung des Markers beispielsweise durch Eindringen der freien Enden des Federdrahts in das Gewebe erfolgt nicht.

Aus der Figur 14 der WO 96/08208 ist eine weitere Vorrichtung zum *in vivo* Setzen von Markern in ein biologisches Gewebe bekannt, bei der eine Vorform eines Markers aus Federdraht so elastisch verformt in einer Applikatorkanüle angeordnet ist, dass sich die freien Enden des Federdrahts beim Freiwerden der Vorform aus der Applikatorkanüle seitlich bewegen und in das Gewebe eindringen, um den Marker zu verankern. Auch bei dieser bekannten Vorrichtung ist die Vorform in der Applikatorkanüle gegenüber dem Marker um eine Achse zusammengelegt, die hier aber quer zu der Applikatorkanüle verläuft, wobei der freie Querschnitt der Applikatorkanüle nur zu geringen Teilen von dem Federdraht ausgefüllt wird. Verglichen mit der Stärke des Federdrahts ist der Innendurchmesser der Applikatorkanüle daher auch hier vergleichsweise groß.

Weiterhin ist aus der Figur 16 der WO 96/08208 eine Vorrichtung zum *in vivo* Setzten von Markern in eine biologisches Gewebe bekannt, bei der in einer speziellen, an ihrem distalen Ende geschlossenen und spitz zulaufenden Applikatoreinrichtung ein Kanal vorgesehen ist, der zunächst vom proximalen Ende der Applikatoreinrichtung zu dem distalen Ende hin geradlinig verläuft und dann mit einer Krümmung vor dem angespitzten distalen Ende seitlich ausmündet. Wenn durch diesen Kanal ein duktiler Draht vom proximalen Ende der Applikatoreinrichtung her durchgeschoben wird, wird dieser beim Durchlaufen der Krümmung am Ende des Kanals in einen helikalen Marker verformt, der seitlich abgesetzt wird, wobei die Achse der Helix des Markers radial zu der Haupterstreckungsrichtung der Applikatoreinrichtung verläuft. Da der Außendurchmesser des helikalen Markers, der in Figur 16 der WO 96/08208 wiedergegeben ist, kaum größer als der Durchmesser der Applikatoreinrichtung ist und da es auch keinen diesbezüglichen Hinweis in der zugehörigen Beschreibung gibt, ist davon auszugehen, dass das zuerst ausgeschobene Ende des duktilen Drahts dieses bekannten Markers nicht in das Gewebe eindringt, sondern dieses vor sich herschiebt. Ein grundsätzlicher Nachteil der hier zuletzt beschriebenen bekannten Vorrichtung zum *in vivo* Setzen von Markern in ein biologisches Gewebe ist, dass die Applikatoreinrichtung ein sehr aufwendig herzustellendes Spezialbauteil darstellt, weil nicht von einer herkömmlichen Kanüle mit einem geradlinig durchgehenden Lumen ausgegangen werden kann. Überdies muss beim Vorschieben des duktilen Drahts durch den gekrümmten Bereich des Kanals in der Applikatoreinrichtung zum Ausbilden der Marker der Verformungswiderstand des duktilen Drahts überwunden werden. Dies erfordert zwar bei dünnen Drähten keine große Kraftanstrengung. Es behindert aber das gefühlvolle Setzen der Marker.

Bei allen Markern bei denen der sie ausbildende Draht frei endet, besteht die Gefahr, dass sie in dem biologischen Gewebe, in das sie gesetzt wurden, wandern; auch, und insbesondere dann, wenn die Enden des Federdrahts in das biologische Gewebe eindringen. Ohne ein Eindringen der Enden des jeweiligen Drahts in das biologische Gewebe ist in jedem Fall eine Wanderbewegung längs des von der Applikatoreinrichtung verursachten Kanals zu befürchten. Relativkräfte zwischen dem jeweiligen Marker und dem ihn umgebenden biologischen Gewebe, die zu einer Wanderbewegung des Markers führen, können durch jedwede aktive oder passive Bewegung des biologischen Gewebes verursacht werden. Durch eine Wanderbewegung eines Markers geht nicht nur dessen Funktion verloren, bestimmte Bereiche des biologischen Gewebes zu markieren, sondern sie kann auch Schmerzen verursachen und je nach der von dem Marker zurückgelegten Wegstrecke auch eine Gefährdung für benachbarte Gewebe, Gefäße und Organe darstellen.

### AUFGABE DER ERFINDUNG

Der Erfindung liegt die Aufgabe zugrunde, eine Vorrichtung zum *in vivo* Setzen von Markern in ein biologisches Gewebe aufzuzeigen, mit der bei geringem konstruktiven und operativen Aufwand Marker setzbar sind, die in dem biologischen Gewebe auch bei stärkeren Bewegungen des Gewebes keine Tendenz zum Wandern aufweisen.

### LÖSUNG

Die Aufgabe wird erfindungsgemäß durch eine Vorrichtung mit den Merkmalen des Patentanspruchs 1 gelöst. Bevorzugte Ausführungsform der neuen Vorrichtung sind in den Unteransprüchen 2 bis 12 beschrieben.

### BESCHREIBUNG DER ERFINDUNG

Bei der neuen Vorrichtung ist die Vorform des Markers in der Applikatorkanüle linear gestreckt, und die Spitze der Vorform beschreibt beim Freiwerden aus der Applikatorkanüle einen tangential in das distale Ende der Applikatorkanüle einmündenden Kreisbogen. Der Marker bzw. die Vorform besteht dabei aus einem Federdraht, der bei der Vorform elastisch verformt ist. Wenn die Spitze der Vorform beim Freiwerden aus der Applikatorkanüle einen Kreisbogen beschreibt, bedeutet dies daher, dass die Vorform durch die in ihr wohnenden elastischen Rückstellkräfte die Kreisbogenform annimmt. Dies geschieht bei ihrem Austreten aus dem distalen Ende der Applikatorkanüle, also in dem Moment, in dem sie durch die Applikatorkanüle nicht mehr linear gestreckt wird. Die Applikatorkanüle kann damit insbesondere auf einer einfachen geraden Kanüle, d.h. einer Hohlnadel basieren. Die Umformung der zunächst linear gestreckten Vorform in den ringförmigen Marker erfolgt ausschließlich durch die elastischen Rückstellkräfte.

Insbesondere wenn der Federdraht des ringförmigen Markers einen Verlauf genau in einer Ebene aufweist und einem Kreisbogen mit konstantem Krümmungsradius folgt, besteht keine Gefahr eines Wandern des Markers, wenn dieser mit der Spitze der Vorform in das biologische Gewebe eindringt und in dem biologischen Gewebe eine Kreisbahn beschreibt. Dabei muss diese Kreisbahn beim Setzen des Markers nicht geschlossen werden. Es ist völlig ausreichend, wenn der Federdraht bei dem ringförmigen Marker einen Kreisbogen von mindestens 270° beschreibt. Günstiger ist es allerdings, wenn dieser Kreisbogen mindestens 330° überspannt, d.h. zumindest nahezu geschlossen ist. Am meisten bevorzugt ist ein ringförmig geschlossener Marker, bei dem sich die Enden des Federdrahts auch überlappen können.

Um das Eindringen der Spitze der Vorform in das biologische Gewebe zu unterstützen, kann die Spitze der Vorform einen schrägen Anschnitt aufweisen. Dies ist aber bei geringeren Drahtstärken des Federdrahts nicht zwingend erforderlich.

Ob die Spitze der Vorform wie gewünscht in das biologische Gewebe eindringt und darin einen Ringkanal ausbildet, in dem der Marker zu liegen kommt, hängt auch davon ab, wie groß der Außendurchmesser des Markers ist. Dieser sollte größer als 3 mm sein. Ein besonders geeigneter Bereich für den Außendurchmesser des Markers liegt zwischen 4,0 und 7,0 mm. Die Drahtstärke des Federdrahts sollte bei kleineren Außendurchmessern des Markers tendenziell kleiner gehalten werden. Sie sollte mindestens 0,2 mm betragen. Bei größeren Drahtstärken im Bereich von 0,3 bis 0,5 mm ist ein schräger Anschnitt des Federdrahts an der Spitze der Vorform sinnvoll, um ihr Eindringen in das Gewebe zu erleichtern.

Der Federdraht, aus dem die Vorform und der Marker ausgebildet sind, muss biologisch verträglich sein. Dem Fachmann sind verschiedene Metalllegierungen zur Anwendung in der Medizin bekannt, auf die diese Anforderung zutrifft. Besonders bevorzugt ist für den Federdraht ein superelastisches Material, wie beispielsweise Nickel-Titan-Legierungen, die als Nitinol gehandelt werden. Superelastisches Material weist einen großen Bereich elastischer Verformbarkeit auf. Dies erleichtert es bei der neuen Vorrichtung, die ringförmigen Marker in linear gestreckte Vorformen zu bringen, ohne den Federdraht plastisch zu verformen, wonach er nicht mehr in die gewünschte Ringform zurückkehren würde. Auch ein Setzen des Federdrahts innerhalb der Applikatorkanüle, das ebenfalls der Rückkehr in die Ringform des Markers entgegen stehen würde, wird durch Verwendung von superelastischem Material für den Marker vermieden.

Die Applikatorkanüle der neuen Vorrichtung kann sehr dünn gehalten werden, da der Federdraht bei der gestreckten Vorform des Makers den freien Querschnitt der Applikatorkanüle im Wesentlichen ausfüllt. Mit anderen Worten muss der freie Querschnitt der Applikatorkanüle nur unwesentlich größer als die Drahtstärke des Federdrahts sein. Auch die von der Vorform auf die Applikatorkanüle ausgeübten elastischen Rückstellkräfte verlangen keine starke Dimensionierung der Applikatorkanüle.

Die Betätigungsvorrichtung der neuen Vorrichtung zum Ausstoßen der Vorform aus dem distalen Ende der Applikatorkanüle kann einen Mandrel in der Applikatorkanüle aufweisen, der mit einem Betätigungselement um die linear gestreckte Länge der Vorform bis gegen einen Anschlag für das Betätigungselement vorschiebbar ist. Das Betätigungselement ist dabei vorzugsweise in Richtung der Applikatorkanüle linear geführt, um ein Ausknicken des Mandrels zu verhindern. Ein Austreten des Mandrels aus dem distalen Ende der Applikatorkanüle ist unerwünscht.

Bei einer bevorzugten Ausführungsform der neuen Vorrichtung verläuft der Kreisbogen, den die Spitze der Vorform beschreibt, in einer radialen Richtung von der Applikatorkanüle weg, wobei diese radiale Richtung am proximalen Ende der Applikatorkanüle markiert ist. Der Operateur kann damit die radiale Richtung gegenüber der Applikatorkanüle vorgeben, in der der jeweilige Marker in das biologische Gewebe gesetzt wird. Beispielsweise kann hierdurch erreicht werden, dass jeder Marker auf einer von einem zu markierenden Tumor abgewandten Seite der Applikatorkanüle in das biologische Gewebe gesetzt wird. Die radiale Richtung des Kreisbogens wird bei der neuen Vorrichtung beispielsweise dadurch vorgegeben, dass die Vorform bezüglich der Richtung ihrer Rückstellkräfte eine definierte Ausrichtung um die Haupterstreckungsrichtung der Applikatorkanüle aufweist und dass am distalen Ende eine Führung für die Spitze der Applikatorkanüle ausgebildet ist, so dass diese Ausrichtung beibehalten bleibt. Eine derartige Führung kann beispielsweise durch einen leichten Schräganschliff des distalen Endes oder eine radiale Ausnehmung am distalen Ende der Applikatorkanüle erreicht werden.

Die Applikatorkanüle kann an ihrem distalen Ende einen Nadelanschliff aufweisen, um mit ihr das Gewebe direkt zu punktieren, in welches der Marker gesetzt werden soll. Vorzugsweise ist aber eine zusätzliche Punktionskanüle zum Aufnehmen der Applikatorkanüle vorgesehen. Dabei ist es besonders bevorzugt, wenn die Applikatorkanüle in einer definierten Relativstellung von der Punktionskanüle aufgenommen wird. Zu dieser definierten Relativstellung gehört eine definierte Lage des distalen Endes der Applikatorkanüle in der und um die Haupterstreckungsrichtung der Punktionskanüle. Insbesondere sollte die Applikatorkanüle im Bereich des Nadelanschliffs der Punktionskanüle enden und eine solche Drehstellung aufweisen, dass der Marker auf der durch den Nadelanschliff kürzeren Seite der Punktionskanüle in das Gewebe gesetzt wird. Die Drehstellung der Applikatorkanüle gegenüber der Punktionskanüle kann an deren proximalen Enden beispielsweise durch eine Steckverbindung aus formschlüssig ineinander greifenden Teilen festgelegt werden.

Um auch bei einer Überwachung des Setzens des Markers mit der neuen Vorrichtung durch Ultraschall die Orientierung des Nadelanschliffs der Punktionskanüle erkennen zu können, kann die Punktionskanüle an ihrem distalen Ende ein Echofeld auf der Seite aufweisen, auf der sie durch ihren Nadelanschliff radial weiter offen, d.h. kürzer ist. Vorzugsweise ist die Lage des Echofelds so gewählt, dass der Marker genau über diesem Echofeld gesetzt wird, so dass seine Lage durch kontrollierte Platzierungen und Orientierungen des Echofelds vorbestimmt werden kann. Insbesondere dann, wenn keine separate Punktionskanüle vorgesehen ist, kann auch die Applikatorkanüle in entsprechender Weise mit einem Echofeld versehen sein.

### KURZBESCHREIBUNG DER FIGUREN

Im Folgenden wird die Erfindung anhand eines in den Figuren dargestellten bevorzugten Ausführungsbeispiels weiter erläutert und beschrieben.
- **Fig. 1**: zeigt eine Punktionskanüle und eine Applikatorkanüle mit Betätigungsvorrichtung einer Vorrichtung zum *in vivo* Setzen von Markern in ein biologisches Gewebe.
- **Fig. 2**: zeigt die Vorrichtung gemäß Fig. 1 im zusammengebauten Zustand mit in die Punktionskanüle eingeschobener Applikatorkanüle.
- **Fig. 3**: zeigt in vergrößerter Darstellung das mit einem Nadelanschliff versehene distale Ende der Punktionskanüle mit dem darin liegenden distalen Ende der Applikatorkanüle in einer Draufsicht.
- **Fig. 4**: zeigt einen Längsschnitt durch das distale Ende der Punktionskanüle und der Applikatorkanüle mit einer darin angeordneten Vorform eines Markers.
- **Fig. 5**: zeigt eine Anfangsphase beim Setzten eines Markers, wobei die Punktionskanüle weggelassen ist.
- **Fig. 6**: zeigt ein vergrößertes Detail von Fig. 5.
- **Fig. 7**: zeigt eine weiter fortgeschrittene Phase des Setzvorgangs gemäß Fig. 5.
- **Fig. 8**: zeigt einen ringförmigen Marker, wie er gemäß den Figuren 5 und 6 gesetzt wurde, wobei hier auch die Punktionskanüle wiedergegeben ist.
- **Fig. 9**: zeigt eine konkrete Ausführungsform des Markers; und
- **Fig. 10**: zeigt eine andere konkrete Ausführungsform des Markers.

### FIGURENBESCHREIBUNG

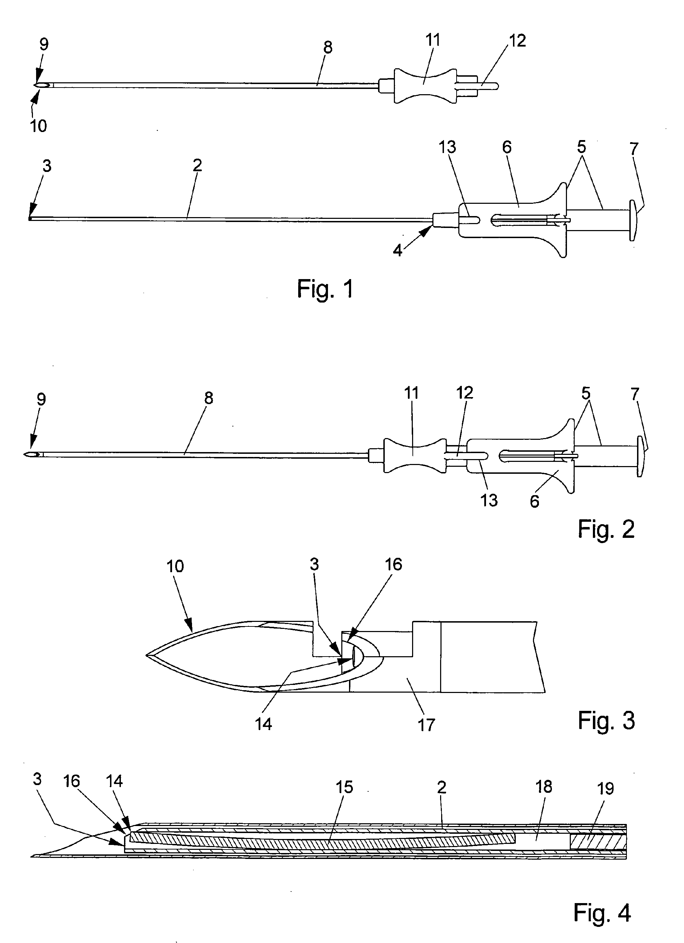

**Fig. 1** zeigt eine Vorrichtung 1 zum *in vivo* Setzen von Markern in ein biologisches Gewebe. Die Marker sind in Fig. 1 nicht zu erkennen. Die Vorform mindestens eines von ihnen befindet sich in einer Applikatorkanüle 2 und liegt in dieser vor deren distalen Ende 3. Am proximalen Ende 4 der Applikatorkanüle 2 ist eine Betätigungsvorrichtung 5 vorgesehen, die einen gegenüber einem Griffstück 6 eindrückbaren Betätigungskolben 7 aufweist. Der Betätigungskolben 7 kann gegenüber dem Griffstück 6 in der axialen Richtung der Applikatorkanüle 2 um ein Maß vorgeschoben werden, das gerade ausreichend ist, um mit einem mit dem Betätigungskolben 7 verbundenen, in der Applikatorkanüle 2 verlaufenden und daher in Fig. 1 nicht sichtbaren Mandrel die Vorform des Markers aus dem distalen Ende 3 der Applikatorkanüle 2 auszustoßen. Weiterhin gehört zu der Vorrichtung 1 gemäß Fig. 1 eine Punktionskanüle 8 mit einem distalen Ende 9 und einem Griffstück 11 an ihrem proximalen Ende. Die Punktionskanüle 8 dient zur Aufnahme der Applikatorkanüle 2, die von dem Griffstück 11 der Punktionskanüle 8 her eingeschoben wird, bis ihr distales Ende 3 im Bereich des distalen Endes 9 der Punktionskanüle 8, wo diese mit einem Nadelanschliff 10 versehen ist, angeordnet ist. Dann schlägt das Griffstück 6 der Applikatorkanüle 2 an ein Griffstück 11 der Punktionskanüle 8 an. Eine Feder 12 am Griffstück 11 der Punktionskanüle 8 und eine Nut 13 im Griffstück 6 der Applikatorkanüle 2 geben die relative Winkelausrichtung der distalen Enden 3 und 9 der Applikatorkanüle 2 und der Punktionskanüle 8 um deren gemeinsame Haupterstreckungsrichtung vor und zeigen die absolute Winkelausrichtung des Nadelanschliffs 10 an.

**Fig. 2** zeigt die Vorrichtung 1 gemäß Fig. 1 im zusammengesetzten Zustand mit in die Punktionskanüle 8 eingeschobener Applikatorkanüle 2. Der Anschlag des Griffstücks 6 der Applikatorkanüle 2 an das Griffstück 11 der Punktionskanüle 8 erfolgt genau dann, wenn das distale Ende 3 der Applikatorkanüle 2 bis in den Bereich des Nadelanschliffs 10 der Punktionskanüle 8 reicht. Gleichzeitig definiert die aus der Feder 12 und der Nut 13 ausgebildete Steckverbindung zwischen den Griffstücken 6 und 11 die Winkelstellung um die gemeinsame Haupterstreckungsrichtung. Dies ist wichtig, weil das distale Ende 3 der Applikatorkanüle 2 um deren Haupterstreckungsrichtung ebenso wenig rotationssymmetrisch ausgebildet ist wie die vor dem distalen Ende 3 in der Applikatorkanüle 2 angeordnete Vorform für einen Marker. Hierauf wird im Zusammenhang mit den weiteren Figuren näher eingegangen werden.

**Fig. 3** zeigt in vergrößerter Darstellung das distale Ende 9 der Punktionskanüle 8 mit dem dort angebrachten Nadelanschliff 10. Im Bereich des Nadelanschliffs 10 ist auch das distale Ende 3 der Applikatorkanüle 2 zu sehen. Hierin ist die Spitze 14 einer Vorform 15 des Markers zu erkennen, weil die Applikatorkanüle 2 an ihrem distalen Ende 3 eine radiale Ausnehmung 16 aufweist, die in dieselbe Richtung weist wie der Nadelanschliff 10. Benachbart dem Nadelanschliff 10 ist ein Echofeld 17 an die Punktionskanüle 8 angeätzt, das es ermöglicht, durch Ultraschalltechniken die Lage des distalen Endes 9 der Punktionskanüle 8 in einem Gewebe und insbesondere dessen Relativlage zu anderen Objekten in dem Gewebe zu erkennen.

**Fig. 4** ist ein Längsschnitt durch das distale Ende 3 der aus der Punktionskanüle 8 und der Applikatorkanüle 2 zusammengesetzten Vorrichtung 1 und zeigt die Vorform 15, von der in Fig. 3 nur die Spitze 14 zu erkennen war. Die Vorform 15 liegt linear gestreckt in dem Lumen 18 der Applikatorkanüle 2. Um diese gestreckte Form zu erreichen, ist die Vorform 15 aus einer Ringform elastisch verformt, so dass in der Vorform 15 Rückstellkräfte wirken, die aber in ihrer Lage gemäß Fig. 4 durch die Wandung der Applikatorkanüle 2 abgestützt werden. Nur die Spitze 14 der Vorform 15 biegt im Bereich der radialen Ausnehmung 16 der Applikatorkanüle 2 schon etwas ab. Zum Setzen des sich aus der Vorform 15 ausbildenden Markers ist der hinter der Vorform 15 in dem Lumen 18 liegende Mandrel 19 mit dem Betätigungskolben 7 gemäß Fig. 1 und 2 vorschiebbar, so dass die Vorform 15 aus dem distalen Ende 3 der Applikatorkanüle 2 ausgestoßen wird.

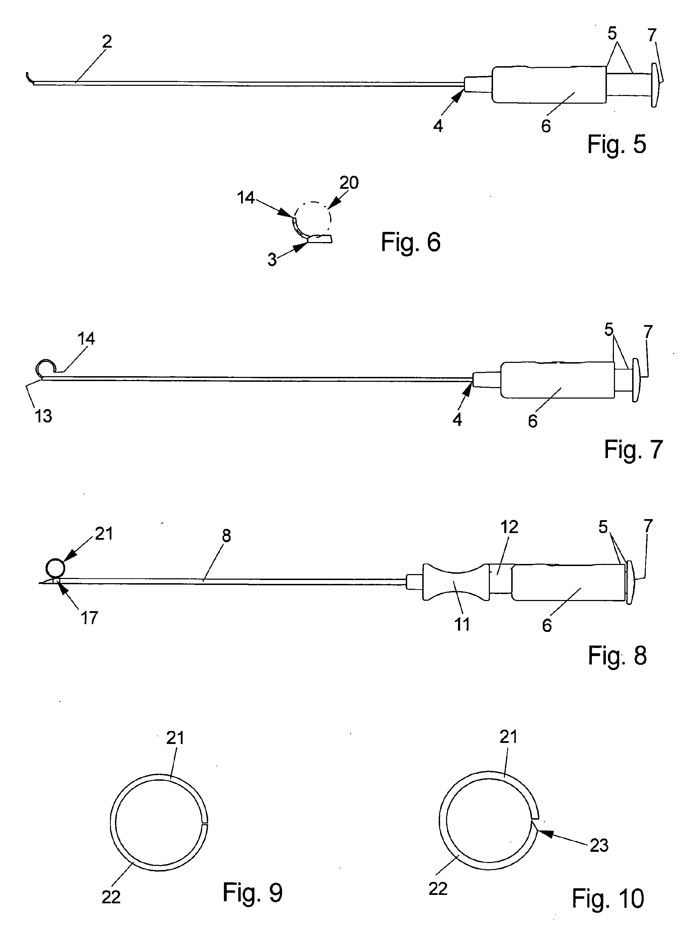

Dieser Vorgang ist in den **Fig. 5** bis **8** dargestellt, wobei in den Fig. 5 bis 7 nur die Punktionskanüle 8 weggelassen ist und Fig. 6 ein vergrößertes Detail von Fig. 5 ist. Die aus dem distalen Ende 3 der Applikatorkanüle 2 austretende Spitze 14 der Vorform 15 beschreibt einen Kreisbogen 20, der tangential in das distale Ende 3 der Applikatorkanüle 2 einmündet, bzw. aus diesem ausmündet. Beim Setzen eines Markers dringt die Spitze 14 in das oberhalb des Nadelanschliffs 10 der Punktionskanüle 8 liegende Gewebe ein. Im Folgenden wird, wie insbesondere aus Fig. 6 abzuleiten ist, von der Spitze 14 ein ringförmiger Kanal in das Gewebe gestochen, in dem der in den Fig. 9 und 10 in zwei Ausführungsformen separat dargestellte Marker 21, der aus der elastischen Rückformung der Vorform 15 resultiert, ortsfest in dem Gewebe angeordnet wird. Der Marker 21 und damit auch seine Vorform 15 besteht aus einem Federdraht 22 der ohne plastische Verformung aus der Ringform des Markers 21 elastisch in die linear gestreckte Form der Vorform 15 verformbar ist. Besonders geeignet für den Federdraht 22 ist superelastisches Material, wie beispielsweise unter dem Handelsnamen Nitinol erhältlich. Die Spitze 14 der Vorform 15 kann, wie in Fig. 10 gezeigt ist, einen schrägen Anschnitt 23 aufweisen, um ihr Eindringen in das dem Nadelanschliff 10 der Punktionskanüle 8 gemäß Fig. 3 gegenüberliegende Gewebe zu unterstützen. Allein aufgrund der typischen geringen Drahtstärke des Federdrahts 22 von 0,2 bis 0,5 mm ist ein Eindringen eines Markers 21 ohne einen besonderen Anschliff, wie er in Fig. 9 gezeigt ist, in das Gewebe möglich. Der Marker 21 kann, wie in beiden Fig. 9 und 10 gezeigt ist, einen geschlossenen Ring ausbilden, bei dem der Federdraht 22 einen Kreisboden von ziemlich genau 360° überspannt, so dass die Spitze 14 direkt an das rückwärtige Ende der Vorform anschließt. Es ist aber auch möglich, dass der von dem Marker 21 beschriebene Ring nicht völlig geschlossen ist oder sich andererseits die Enden des Federdrahts 22 überlappen. Vorzugsweise verläuft der Federdraht 22 bei dem Marker 21 aber in eine Ebene, er weist also keine Steigung wie bei einer Helix auf. Der Durchmesser des Markers 21 beträgt typischerweise einige wenige Millimeter. Konkrete Ausführungsbeispiele weisen einen Außendurchmesser des Markers 21 von 5,4 mm bei Drahtstärken des Federdrahts 22 von 0,3 oder 0,4 mm auf. Die Ebene, in der der Kreisbogen 20 verläuft, dem die Spitze 14 der Vorform 15 beim Vorschieben aus dem distalen Ende 3 der Applikatorkanüle 2 folgt, verläuft radial zu der Haupterstreckungsrichtung der Applikatorkanüle 2. Die Drehstellung dieser Ebene um die Haupterstreckungsrichtung wird durch die Ausnehmung 16 am distalen Ende 3 der Applikatorkanüle 2 festgelegt. Bei Verwendung der Applikatorkanüle 2 zusammen mit der Punktionskanüle 8 wird der jeweilige Marker gemäß Fig. 7 genau über dem Echofeld 17 gesetzt. Mit anderen Worten kann die Position des Markers 21 durch die Ausrichtung des Echofelds 17 in dem biologischen Gewebe vorgegeben und so durch übliche Ultraschalltechniken überwacht werden.

### BEZUGSZEICHENLISTE

- 1: Vorrichtung
- 2: Applikatorkanüle
- 3: distales Ende
- 4: proximales Ende
- 5: Betätigungsvorrichtung
- 6: Griffstück
- 7: Betätigungskolben
- 8: Punktionskanüle
- 9: distales Ende
- 10: Nadelanschliff

- 21: Marker
- 22: Federdraht
- 23: Anschnitt

- 11: Griffstück
- 12: Feder
- 13: Nut
- 14: Spitze
- 15: Vorform
- 16: Ausnehmung
- 17: Echofeld
- 18: Lumen
- 19: Mandrel
- 20: Kreisbogen

## Patentansprüche

1. Vorrichtung zum *in vivo* Setzen von Markern in ein biologisches Gewebe mit einer ein offenes distales Ende aufweisenden Applikatorkanüle, in der eine elastisch verformte Vorform mindestens eines ringförmigen Markers aus Federdraht angeordnet ist, und mit einer Betätigungseinrichtung, um die Vorform aus dem distalen Ende der Applikatorkanüle auszustoßen, wobei sich die Vorform beim Freiwerden aus der Applikatorkanüle durch elastische Rückstellkräfte zu dem Marker rückformt, **dadurch gekennzeichnet, dass** die Vorform (15) in der Applikatorkanüle (2) linear gestreckt ist und dass die Spitze (14) der Vorform (15) beim Freiwerden aus der Applikatorkanüle (2) einen tangential in das distale Ende (3) der Applikatorkanüle (2) einmündenden Kreisbogen (20) beschreibt.

2. Vorrichtung nach Anspruch 1, **dadurch gekennzeichnet, dass** die Applikatorkanüle (2) gerade ist.

3. Vorrichtung nach einem der Ansprüche 1 bis 2, **dadurch gekennzeichnet, dass** der Federdraht (22) bei dem ringförmigen Marker (21) einen Verlauf in einer Ebene aufweist.

4. Vorrichtung nach einem der Ansprüche 1 bis 3, **dadurch gekennzeichnet, dass** der Federdraht (22) bei dem ringförmigen Marker (21) einen Kreisbogen von mindestens 270°, mehr bevorzugt von mindestens 330° und am meisten bevorzugt von mindestens 360° überspannt.

5. Vorrichtung nach einem der Ansprüche 1 bis 4, **dadurch gekennzeichnet, dass** der Außendurchmesser des Markers (21) größer als 3 mm ist und dass der Federdraht (22) eine Drahtstärke von mindestens 0,2 mm aufweist.

6. Vorrichtung nach einem der Ansprüche 1 bis 5, **dadurch gekennzeichnet, dass** die Spitze (14) der Vorform (15) einen schrägen Anschnitt (23) aufweist.

7. Vorrichtung nach mindestens einem der Ansprüche 1 bis 6, **dadurch gekennzeichnet, dass** der Federdraht (22) aus superelastischem Material besteht.

8. Vorrichtung nach einem der Ansprüche 1 bis 7, **dadurch gekennzeichnet, dass** der Federdraht (22) den freien Querschnitt der Applikatorkanüle (2) im Wesentlichen ausfüllt.

9. Vorrichtung nach mindestens einem der Ansprüche 1 bis 8, **dadurch gekennzeichnet, dass** die Betätigungsvorrichtung (5) einen Mandrel (19) in der Applikatorkanüle (2) aufweist, der mit einem Betätigungselement (7) um die linear gestreckte Länge der Vorform (15) bis gegen einen Anschlag für das Betätigungselement (7) vorschiebbar ist.

10. Vorrichtung nach einem der Ansprüche 1 bis 9, **dadurch gekennzeichnet, dass** der Kreisbogen (20), den die Spitze (14) der Vorform (15) beschreibt, in einer radialen Richtung von der Applikatorkanüle (2) weg verläuft, die am proximalen Ende (4) der Applikatorkanüle (2) markiert ist.

11. Vorrichtung nach einem der Ansprüche 1 bis 10, **dadurch gekennzeichnet, dass** eine Punktionskanüle (8) zum Aufnehmen der Applikatorkanüle (2) in einer definierten Winkelstellung der Applikatorkanüle (2) gegenüber der Orientierung eines Nadelanschliffs (10) am distalen Ende (9) der Punktionskanüle (8) vorgesehen ist.

12. Vorrichtung nach Anspruch 11, **dadurch gekennzeichnet, dass** die Punktionskanüle (8) an ihrem distalen Ende (9) ein Echofeld (17) auf der Seite aufweist, auf der sie durch ihren Nadelanschliff (10) radial weiter offen ist.
